# EUROPEAN PATENT SPECIFICATION

(11) **EP 2 580 594 B1**
(45) Date of publication and mention of the grant of the patent: **18.04.2018**
(21) Application number: 11735930.7
(22) Date of filing: 08.06.2011
(51) Int. Cl.: G01N 33/574

(54) **A METHOD OF PREDICTING CLINICAL OUTCOMES FOR MELANOMA PATIENTS USING CIRCULATING MELANOMA CELLS IN BLOOD**
VERFAHREN ZUR VORHERSAGE DER KLINISCHEN ERGEBNISSE FÜR MELANOMPATIENTEN MIT ZIRKULIERENDEN MELANOMZELLEN IM BLUT
MÉTHODE UTILISANT DES CELLULES DE MÉLANOME EN CIRCULATION DANS LE SANG POUR PRÉDIRE DES RÉSULTATS CLINIQUES DE PATIENTS PRÉSENTANT UN MÉLANOME

(30) Priority: 08.06.2010 US 352441 P
(43) Date of publication of application: 17.04.2013
(73) Proprietor: Menarini Silicon Biosystems, Inc., San Diego, CA 92121 (US)
(72) Inventor: RAO, Galla, Chandra, Princeton Junction NJ 08550 (US); CONNELLY, Mark, C., Doylestown PA 18901 (US)
(74) Representative: Goodfellow, Hugh Robin
(86) International application number: PCT/US2011/039596
(87) International publication number: WO 2011/156468

(56) References cited:
- US-A1- 2009 136 946
- RAO CHANDRA ET AL: "Automated enumeration and characterization of circulating melanoma cells in blood", PROCEEDINGS OF THE ANNUAL MEETING OF THE AMERICAN ASSOCIATION FOR CANCER RESEARCH, vol. 49, 1 April 2008 (2008-04-01), page 874, XP009143620, ISSN: 0197-016X
- ULMER A ET AL: "Immunomagnetic Enrichment, Genomic Characterization, and Prognostic Impact of Circulating Melanoma Cells", CLINICAL CANCER RESEARCH, vol. 10, no. 2, 15 January 2004 (2004-01-15), pages 531-537, XP55008451, ISSN: 1078-0432, DOI: 10.1158/1078-0432.CCR-0424-03
- STEEN S ET AL: "Circulating tumor cells in melanoma: a review of the literature and description of a novel technique", PROC (BAYL UNIV MED CENT), vol. 21, no. 2, 1 January 2008 (2008-01-01), pages 127-132, XP55007083,
- MEDIC S ET AL: "Molecular markers of circulating melanoma cells", PIGMENT CELL RESEARCH, vol. 20, no. 2, 1 April 2007 (2007-04-01), pages 80-91, XP009140695, ISSN: 0893-5785, DOI: 10.1111/J.1600-0749.2006.00356.X [retrieved on 2006-12-20]

## Description

### Background

Treatment of advanced melanoma is complicated by its heterogeneous histopathology and changes in make-up that accumulate during tumor progression. The enumeration and characterization of circulating tumor cells in patients with either metastatic breast or colorectal cancer have been shown to provide independent prognostic and predictive information that is clinically significant and can be used to monitor patient management.

Circulating tumor cells (CTCs) have been shown to be a critical link between primary cancer, a disease stage at which cure is possible, and metastatic disease, which continues to be the leading cause of death for most malignancies. Clinical studies have shown that CTCs are a powerful prognostic and predictive biomarker in metastatic breast cancer, and similar findings have been reported in prostate cancer and colorectal cancer. These data show that CTCs are representative of the underlying biology driving metastatic cancer and suggest that further cellular and molecular analyses of these cells can reveal new insights into molecular regulation of metastasis and response to therapy.

Methods to capture, enumerate, and characterize CTCs have been modified to capture, enumerate, and characterize circulating melanoma cells (CMCs) in a patient's blood. See Automated Enumeration and Characterization of Circulating Melanoma Cells in Blood, U.S. Pat. Application No. 12/254188, filed 10/20/2008. Even though CMCs were captured, enumerated and characterized by this method, the predictive value, with respect to the short term survival of patients with metastatic melanoma was unknown. This invention offers a method of predicting overall survival for patients with metastatic melanoma.

US 2009/0136946 discusses a method for enumerating circulating cancer cells, but it does not disclose a step of predicting the overall survival of a patient by whether the number of detected circulating melanoma cells is greater than or less than two. Ulmer et al., 2004 discusses a method of analysis of circulating melanoma cells that uses 50ml of blood. Steen et al, 2008, provides a review of the literature about circulating tumor cells in melanoma and a description of a new technique. Chandra et al, 2008, relates to the automated enumeration and characterization of circulating melanoma cells in blood. Medic et al, 2007, relates to molecular markers of circulating melanoma cells.

### Brief Description of the Drawings

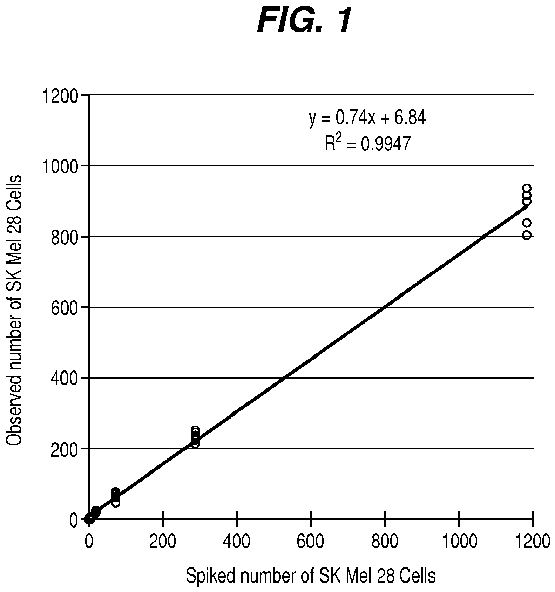

**Figure 1****.** Recovery of known numbers of spiked SK-Mel 28cells from whole blood. SK-Mel28 cells spiked into the healthy donor samples (i.e., 0, 5, 18, 72, 280 and 1183 cells) were spiked into 7.5 mL of blood from five healthy donors on each of 2 days with a total of 5 different samples at each cell level. The number of cells spiked is plotted versus the observed number of cells recovered.

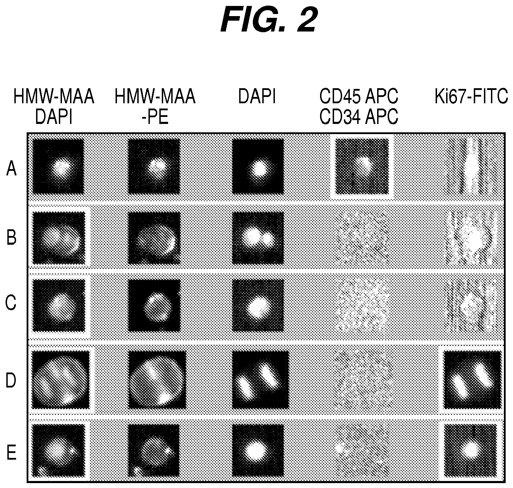

**Figure 2****.** Rows A - E represent objects that were identified by the CellTracks Analyzer II® software as objects having both DAPI and PE signal in a sample from a melanoma patient. From right to left, the thumbnail images represent the Ki67 FITC signal, the CD45 and/or CD34 APC signal, the DAPI signal, the HMW-MAA PE signal and the overlay of DAPI (purple) and HMW-MAA (green) signal. The cell in Row A is excluded as a melanoma cell as it expresses CD45 and/or CD34, the cell in Rows B and C is classified as melanoma cells that do not express Ki67 and the cell in Rows D and E is classified as melanoma cells that do express Ki67.

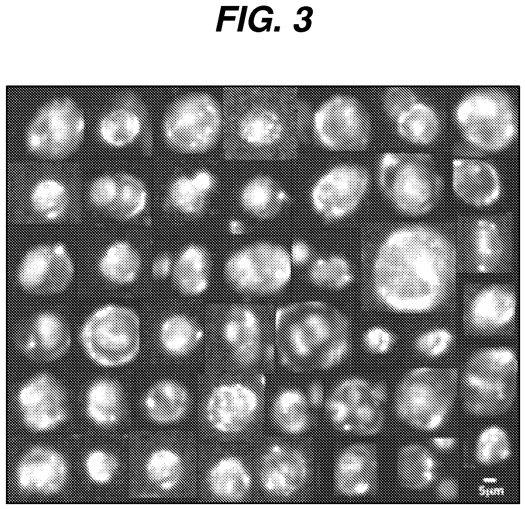

**Figure 3****.** Gallery of typical CMC images from the CellTracks Analyzer II® obtained from 7.5 mL of blood from melanoma patients.

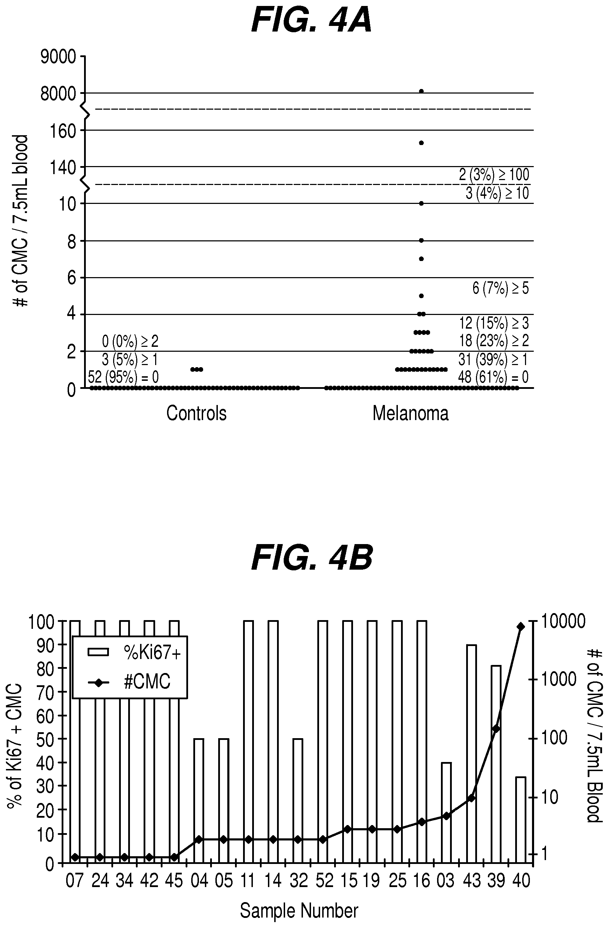

**Figure 4****.** Prevalence of CMC in 7.5 mL of blood of 55 healthy donors, 79 samples from 44 metastatic melanoma patients, Panel A and the percentage of Ki67 expressing CMCs in 19 samples from 16 melanoma patients, Panel B.

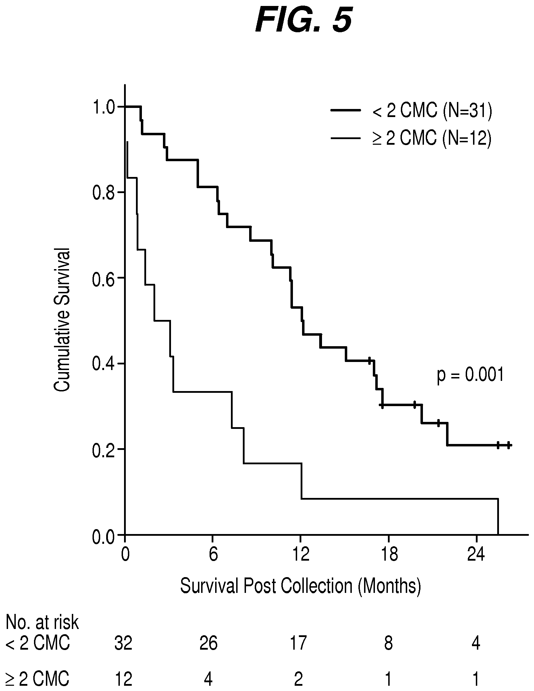

**Figure 5****.** Kaplan-Meier estimates of probabilities of Overall Survival in patients with metastatic melanoma for those with <2 Circulating Melanoma Cells per 7.5 ml of whole blood and those in the group with ≥2 Circulating Melanoma Cells in 7.5 ml of whole blood. OS times were calculated from the time of each blood draw. Median survival is 12.1 months for the group with < 2 CMC versus 2.0 months for people with ≥ 2 CMC (P=0.001 by the log-rank test; hazard ratio of death in patients with ≥2 cells per 7.5 ml, 3.2).

### Detailed Description of the Invention

The invention provides a method of predicting overall survival for patients with metastatic melanoma in a 7.5 mL blood sample which has been obtained from a patient with metastatic melanoma, said sample comprising a mixed cell population suspected of containing circulating melanoma cells; wherein the method comprises:(a) enriching a fraction of said specimen, by immunomagnetic enrichment using an externally applied magnetic field to separate paramagnetic particles coupled to CD146 antibodies, to the substantial exclusion of other populations, said fraction containing said circulating melanoma cells; (b) confirming structural integrity of said circulating melanoma cells to be intact, wherein said structural integrity is determined by a nucleic acid dye and a monoclonal antibody specific for High Molecular Weight Melanoma Associated Antigen, and further containing CD45 and CD34 to exclude co-enriched leukocytes and circulating endothelial cells; (c) analyzing said circulating melanoma cells; wherein said analyzing correlates disease progression; (d) evaluating the number of circulating melanoma cells in said blood sample; wherein if the number is greater than or equal to 2 predicting that the patient's overall survival will be no more than two months and wherein if the number of circulating melanoma cells is less than two, predicting that the patient's overall survival will be twelve months.

As used herein the term "enriching" means isolating CMCs from the blood sample of step (a). Methods of enriching include using anti CD146 coupled to magnetic particles to capture cells from the blood sample. The term "confirming" means determining whether the isolated cells are CMCs or other cellular components. Methods of confirming include staining the CMCs with different fluorescently labeled monoclonal antibodies and the antibodies are CD45 & CD34 to exclude leukocytes and endothelial cells, and high molecular weight melanoma associated antigen, HMW-MAA, to identify melanoma cells. The term "analyzing" means evaluating the captured CMCs to determine if the CMCs express a variety of melanoma specific markers such as HMW-MAA, MART-1 (Melanoma antigen recognized by T-cells) and other markers such as Ki-67. The preferred method of analyzing means determining if the CMCs express Ki-67 and/or HMW-MAA. The term "evaluating" means determining how many CMCs are in the sample and using methods which include, but are not limited to, automated image analysis. The preferred method of evaluating is using CellTracks Analyzer II®.

The invention is demonstrated by the following methods and examples. These examples and methods are not intended to limit the scope of the invention.

### Examples

The following methods are provided to facilitate the practice of the present inventions.

**Patients and Blood Collection.** Blood was drawn from healthy volunteers and patients with malignant melanoma into evacuated 10-mL blood CellSave preservative blood draw tube (Veridex LLC, Raritan, NJ) and processed within 72 hours.

The patients were all enrolled from the Department of Medical Oncology of the University of Oxford at the Churchill Hospital using a research ethics committee approved protocol. All patients provided written informed consent. Forty-four patients were enrolled, 25 males and 19 females, and their age ranged from 31-81 (mean 59). At the time of first blood draw 39/44 (86%) had metastatic disease and 5 patients had unresected stage III disease. 38/44 (78%) of patients with metastatic disease had visceral disease, 5/44 (11%) had no visceral involvement and for 1 patient the metastatic sites were not recorded. Median duration of follow up was 10.1 months. Blood was always drawn from cancer patients either before or a minimum of 7 days after the administration of intravenous therapy. Fifty-five healthy volunteers were included as controls and had no known illness or fever at the time of draw and no history of malignant disease.

**Cell Culture and Cell Spiking.** The melanoma cell line SK-Mel28 was cultured in flasks containing RPMI 1640 supplemented with 10% fetal calf serum and subsequently harvested without trypsinization. The cell suspensions were only used when their viability as assessed by trypan blue exclusion exceeded 90%. To determine the actual cell number, 200 µL of buffer and 20 µL of fluorescent beads (Beckman-Coulter, Inc., Miami, FL) containing approximately 20,000 total beads were added to a 50 µL aliquot of the SK-Mel28 cells. The SK-Mel28 cells were stained with anti HMW-MAA conjugated to PE for the detection. Duplicate tubes containing beads only were run on a flow cytometer (FACSCalibur; BD Biosciences, San Jose, CA) until 100% of the sample was aspirated. This provided an accurate estimate of the number of beads present in 20 µL. The experimental tubes were then tested in triplicate on the flow cytometer until 10,000 beads were counted in each tube. The number of SK-Mel28 cells was determined using the known number of beads per unit volume.

**Sample Preparation.** 7.5 mL of blood is transferred to 15 mL CellTracks® AutoPrep® sample tubes and mixed with 6.5 mL of buffer, centrifuged at 800g for 10 minutes, and then placed on the CellTracksAutoprep® (Veridex LLC) for automated sample preparation. Reagents were optimized for capture and detection of melanoma cells and consisted of ferrofluids coated with CD146 antibodies to immunomagnetically enrich both melanoma cells and endothelial cells, a capture enhancement reagent to maximize the capture efficiency, a phycoerythrin-conjugated antibody that binds to the High Molecular Weight Melanoma Associate Antigen (HMW-MAA) (clone 9.2.27, Veridex LLC) to identify melanoma cells, a mixture of two allophycocyanine conjugated antibodies to identify leukocytes (CD45, clone HI30, Veridex LLC) and endothelial cells (CD34, clone 581, BD Biosciences), a FITC conjugated antibody identifying the Ki-67 protein (clone B56, BD Biosciences, San Jose, CA), the nuclear dye 4',6-diamidino-2-phenylindole (DAPI) to identify nucleated cells and buffers to wash, permeabilize, and resuspend the cells. In the final processing step, the cells were resuspended in the MagNest® Cell Presentation Device (Veridex LLC). The magnetic field generated by the MagNest device causes the magnetically labeled cells to distribute uniformly over the analysis surface of the cartridge, ready for analysis using the CellTracks Analyzer II®.

**Sample Analysis.** The MagNest is placed on the CellTracks AnalyzerII®, a four-color semi-automated fluorescence microscope. Image frames covering the entire surface of the cartridge for each of the four fluorescent filter cubes are captured. Images that contain PE as well as DAPI positive events are presented in a gallery for classification of the events by the user based on cell fluorescence and morphology. The criteria for an object to be defined as a melanoma cell include round to oval morphology, a visible nucleus (DAPI positive), positive staining for HMW-MAA and negative staining for CD45 and CD34. The melanoma cells were divided in KI67+ and Ki67-cells. Results of cell enumeration are always expressed as the number of cells per 7.5 mL of blood.

**Accuracy, Sensitivity, and Linearity of Melanoma Cell Detection.** For accuracy, linearity, and sensitivity experiments, SK-Mel28 cells were spiked into 7.5 mL of blood collected into CellSave Preservative Tubes at 6 different levels of cells (0, 5, 18, 72, 280 and 1183). The exact number of cells spiked into blood was determined by flow cytometry. The samples were processed 24 hours after spiking the blood on a CellTracks AutoPrep® and analyzed with a CellTracks Analyzer II®. Sample testing was performed over two different days with a total of 5 different samples at each cell level.

### Statistical Analysis

The primary endpoint was overall survival, measured as the time from the sample date to date of death from any cause. Patients who were lost to follow-up or still alive at the end of study were censored at the last date they were known to be alive or at the end of study date. If there were multiple samples per patient, the last sample was used for survival analysis. Overall survival was calculated using the Kaplan-Meier method and a survival plot was generated. Cox regression models were used to determine hazard ratios (HR) of death. Results were analyzed in SPSS 16.0 (SPSS Inc., Chicago, IL, USA).

### Example 1

### Recovery of spiked tissue culture melanoma cell line (SK-Mel28)

In this example, the assay performance using whole blood spiked with SK-Mel28 cells is described. The protocol used for this study was as follows. Whole blood was drawn into CellSave Tubes from healthy volunteers and spiked with tissue culture melanoma SK-Mel28 cells. Varying numbers of SK-Mel28 cells were spiked into blood, and recovery was measured. The expected number of SK-Mel28 cells spiked into the healthy donor samples (*i.e.,* 0, 5, 18, 72, 280 and 1183 cells) plotted against the actual number of SK-Mel28 cells observed in the samples is shown in **Figure 1****,** and results are summarized in **Table 1.** Mean recovery of spiked cells was 88%, with recovery of 74% at the highest spike versus 88% at the 5 SK-Mel28 spike. Pearson R² correlation was 0.99. As expected, the coefficient of variation (CV) increased as the number of cells spiked decreased, ranging from 7% at the 1,183-cell spike to 31% at the 5-cell spike. The recovery of SK-Mel28 cells ranged from 64-120% and did not decrease with lower cell numbers.

**Table 1**

| Method accuracy measured by recovery of SK-Mel 28 cells spiked into 7.5 mL blood of five healthy donors | | | | | | |
|---|---|---|---|---|---|---|
| | Observed CMC Count | | | %Recovery | | |
| Expected CMC count | Average | SD | 95%I | Average | 95%CI | %CV |
| 0 | 0 | 0 | 0 | 0 | 0 | 0 |
| 5 | 4 | 1 | 3-5 | 88 | 64-112 | 31 |
| 18 | 20 | 2 | 18-22 | 110 | 100-120 | 10 |
| 72 | 63 | 11 | 53-73 | 87 | 74-100 | 17 |
| 287 | 234 | 15 | 221-247 | 81 | 77-85 | 6 |
| 1183 | 880 | 56 | 831-929 | 74 | 70-78 | 7 |

**Identification of Circulating Melanoma Cells** Thumbnail images of an overlay of HMW-MAA PE and HMW-MAA PE, DAPI, CD45/CD34 APC and Ki67 are presented to the operator for review. The presence of a nucleus, expression of HMW-MAA, cellular morphology, and a lack of CD45 or CD34 expression are the required characteristics of a CMC. **Figure 2** shows 6 events from one melanoma patient that are presented to the reviewer. **Panel A** shows a cell staining with DAPI and HMW-MAA but also with CD34 and or CD45 and is thus not classified as a CMC. **Panels B, C, D** and **E** show cells staining with DAPI and HMW-MAA but not with CD34 or CD45 and are classified as CMCs. The CMC in **Panels B and C** do not express Ki67 whereas the CMC in **Panels D** and **E** do. Note that the CMC in **Panel B** contains two nuclei and does not stain with Ki67 whereas the CMC in **Panel D** appears to be actively dividing and indeed and indeed expresses Ki67. The size of the CMCs and their nuclear to cytoplasmic ratio vary greatly between CMCs within and between melanoma patients. **Figure 3** shows a gallery of CMC images from different patients with characteristically a round to oval shape and an intact nucleus. Cellular sizes varied over a wide range from 4 µm to 30 µm. Small cell clusters and multinucleated CMCs were also observed.

### Example 2

### Frequency of circulating melanoma cells in healthy volunteers and melanoma patients

**In this example, the frequency of circulating melanoma cells in healthy volunteers and melanoma patients is described.** CMCs were enumerated in 55 blood samples from healthy donor and 79 samples from 44 patients with metastatic melanoma. **Figure 4****, Panel A** shows the number of CMCs detected in 7.5 mL of blood of the control group and the patients. Assessment of Ki67 expression was determined in 19 samples from 17 patients in whom CMCs were detected. The percentage of Ki67+ CMCs ranged from 34 to 100% with a mean of 84% (SD25). **Panel B** of **Figure 4** shows the Ki67 expression and the number of CMCs detected in these samples. In the 55 healthy donors three cells were classified as CMCs and all three did not express Ki67.

### Example 3

### Circulating Melanoma Cells and Overall Survival in Melanoma Patients.

None of the individuals in the control group had 2 or more CMCs detected and this cut-off was chosen to discriminate between patient groups. Mean OS time for those patients with <2 CMCs was 12.1 months (95% CI 9.7. to 14.4) and was significantly longer than the median OS time for those patients with ≥2 CMCs, 2.0 months (95% CI 0 . to 4.9) (**Figure 5**). Logrank p was 0.001. Hazard ratio of death was 3.2 (95% CI 1.6-6.5) by Cox Regression. The four patients that died within 1 month after blood draw had relatively high numbers of CMCs (2, 8, 10 and 8043 CMC/7.5ml).

## Claims

1. A method of predicting overall survival for patients with metastatic melanoma in a 7.5 mL blood sample which has been obtained from a patient with metastatic melanoma, said sample comprising a mixed cell population suspected of containing circulating melanoma cells; wherein the method comprises:
(a) enriching a fraction of said specimen, by immunomagnetic enrichment using an externally applied magnetic field to separate paramagnetic particles coupled to CD146 antibodies, to the substantial exclusion of other populations, said fraction containing said circulating melanoma cells;
(b) confirming structural integrity of said circulating melanoma cells to be intact, wherein said structural integrity is determined by a nucleic acid dye, and a monoclonal antibody specific for High Molecular Weight Melanoma Associated Antigen, and further containing CD45 and CD34 antibodies to exclude co-enriched leukocytes and circulating endothelial cells;
(c) analyzing said circulating melanoma cells; wherein said analyzing correlates disease progression;
(d) evaluating the number of circulating melanoma cells in said blood sample
wherein if the number is greater than or equal to 2, predicting that the patient's overall survival will be no more than two months, and
wherein if the number of circulating melanoma cells is less than two, predicting that the patient's overall survival will be twelve months.

2. The method as claimed in claim 1, wherein said structural integrity is determined by a procedure selected from the group consisting of immunocytochemical procedures, FISH procedures, flow cytometry procedures, image cytometry procedures, and combinations thereof.

3. The method as claimed in claim 1, wherein FITC labeled anti-Ki67 is added to determine the proportion of circulating melanoma cells in active cell cycle within the circulation.

## Patentansprüche

1. Verfahren zur Vorhersage des Gesamtüberlebens für Patienten mit metastatischem Melanom in einer 7,5 mL Blutprobe, die von einem Patienten mit metastatischem Melanom erhalten wurde, wobei die Probe eine gemischte Zellpopulation umfasst, die vermutlich zirkulierende Melanomzellen enthält;
wobei das Verfahren umfasst:
(a) Anreichern einer Fraktion des Präparats durch immunomagnetische Anreicherung unter Verwendung eines von außen angelegten Magnetfeldes, um an CD146-Antikörper gekoppelte paramagnetische Partikel unter wesentlichem Ausschluss anderer Populationen abzutrennen, wobei die Fraktion die zirkulierenden Melanomzellen enthält;
(b) Bestätigen, dass die strukturelle Integrität der zirkulierenden Melanomzellen intakt ist, wobei die strukturelle Integrität durch einen Nukleinsäurefarbstoff und einen monoklonalen Antikörper bestimmt wird, der für Melanomassoziiertes Antigen mit hohem Molekulargewicht spezifisch ist, und wobei ferner CD45- und CD34-Antikörper enthalten sind, um mitangereicherte Leukozyten und zirkulierende Endothelzellen auszuschließen;
(c) Analysieren von zirkulierenden Melanomzellen; wobei das Analysieren mit der Progression der Erkrankung korreliert;
(d) Auswerten der Anzahl der zirkulierenden Melanomzellen in der Blutprobe,
wobei, wenn die Anzahl größer als oder gleich 2 ist, vorhergesagt wird, dass das Gesamtüberleben des Patienten nicht mehr als zwei Monate betragen wird, und wobei, wenn die Anzahl der zirkulierenden Melanomzellen kleiner als zwei ist, vorhergesagt wird, dass das Gesamtüberleben des Patienten zwölf Monate sein wird.

2. Verfahren nach Anspruch 1, wobei die strukturelle Integrität nach einem Verfahren bestimmt wird, das ausgewählt ist aus der Gruppe bestehend aus immunozytochemischen Verfahren, FISH-Verfahren, Durchflusszytometrieverfahren, Bildzytometrieverfahren und Kombinationen davon.

3. Verfahren nach Anspruch 1, wobei FITC-markiertes Anti-Ki67 zugefügt wird, um den Anteil an zirkulierenden Melanomzellen in aktivem Zellzyklus innerhalb der Zirkulation zu bestimmen.

## Revendications

1. Méthode de prédiction de la survie globale de patients atteints de mélanome métastatique dans un échantillon sanguin de 7,5 mL qui a été obtenu auprès d'un patient atteint de mélanome métastatique, ledit échantillon comprenant une population de cellules mixte soupçonnée de contenir des cellules de mélanome en circulation ; où la méthode comprend :
(a) l'enrichissement d'une fraction dudit échantillon par enrichissement immunomagnétique en utilisant un champ magnétique appliqué par voie externe pour séparer des particules paramagnétiques couplées à des anticorps CD146, en excluant substantiellement les autres populations, ladite fraction contenant lesdites cellules de mélanome en circulation ;
(b) la confirmation de l'intégrité structurelle desdites cellules de mélanome en circulation, où ladite intégrité structurelle est déterminée par un colorant de type acide nucléique, est un anticorps monoclonal spécifique de l'antigène associé au mélanome de masse moléculaire élevée, et contenant en outre des anticorps CD45 et CD34 pour exclure les leucocytes co-enrichis et les cellules endothéliales en circulation ;
(c) l'analyse desdites cellules de mélanome en circulation ; où ladite analyse est corrélée à la progression de la maladie ;
(d) l'évaluation du nombre de cellules de mélanome en circulation dans ledit échantillon sanguin
où si le nombre est supérieur ou égal à 2, il est prédit que la survie globale du patient ne sera pas supérieure à 2 mois, et où si le nombre de cellules de mélanome en circulation est inférieur à deux, il est prédit que la survie globale du patient sera de 12 mois.

2. Méthode selon la revendication 1, où ladite intégrité structurelle est déterminée par une procédure choisie dans le groupe constitué par les procédures immunocytochimiques, les procédures FISH, les procédures de cytométrie en flux, les procédures de cytométrie par imagerie et leurs combinaisons.

3. Méthode selon la revendication 1, où l'anti-Ki67 marqué FITC est ajouté pour déterminer la proportion de cellules de mélanome en circulation dans le cycle de cellules actives de la circulation.
